Europäisches Patentamt

European Patent Office

Office européen des brevets

(11) Publication number: **0 415 794 A1**

# EUROPEAN PATENT APPLICATION

(21) Application number: **90309570.1**

(22) Date of filing: **31.08.90**

(51) Int. Cl.⁵: **A61K 39/39**

(30) Priority: **01.09.89 GB 8919819**

(43) Date of publication of application:
**06.03.91 Bulletin 91/10**

(84) Designated Contracting States:
**AT BE CH DE DK ES FR GB GR IT LI LU NL SE**

(71) Applicant: **COOPERS ANIMAL HEALTH LIMITED**
**Berkhamsted Hill**
**Berkhamsted Hertfordshire HP4 2QE(GB)**

(72) Inventor: **Mackenzie, Neill Moray**
**Coopers Animal Health Limited,**
**Berkhamsted Hill**
**Berkhamsted, Hertfordshire(GB)**
Inventor: **O'Sullivan, Angela Marie**
**Coopers Animal Health Limited,**
**Berkhamsted Hill**
**Berkhamsted, Hertfordshire(GB)**

(74) Representative: **Rollins, Anthony John et al**
**Group Patents & Agreements The Wellcome**
**Research Laboratories Langley Court**
**Beckenham Kent BR3 3BS(GB)**

(54) Complexes having adjuvant activity.

(57) "Empty" iscom matrices, ie. formed without an antigen, have been found to provide an adjuvant formation for a separate antigen in a vaccine formulation, the antigen being associated with a bacterium or mycoplasma.

It has also been found that these and conventional iscoms can be formed without removing the solubilising agent used for the antigen.

In each case, the iscom can be 3-dimensional or, if formed without phospholipid, 2-dimensional.

The glycoside is preferably Quil A and the sterol is preferably cholesterol.

EP 0 415 794 A1

## COMPLEXES HAVING ADJUVANT ACTIVITY

The present invention relates to complexes having adjuvant activity, the complexes comprising lipid and at least one glycoside, and to novel glycosides for incorporation in said complexes.

Prophylactic immunisation of animals against microbes comprises administration of an antigen derived from the microbe in conjunction with a material that increases the antibody and/or cell-mediated immune response of the antigen in the animal. This material is known as an adjuvant. The only adjuvants currently authorised for use in humans or pigs in many countries are aluminium hydroxide and aluminium phosphate. Although these adjuvants are sufficient for many vaccines, studies have shown that Freund's complete adjuvant (FCA) or Quil A (also known as saponin) are often more efficacious in eliciting antibody response and cell mediated immunity but unfortunately these adjuvants may cause the animals to react adversely to vaccination.

EP-A-0 109 942 and EP-A-0 180 564 describe immunogenic complexes formed between glycosides, such as triterpenoid saponins (particularly Quil A), and antigens which contain a hydrophobic region. These immuno-stimulating complexes have been given the name "iscoms". The amount of Quil A in an iscom can be about 10 to 100 times lower than when Quil A is mixed with the antigen to produce the same antigenic effect. Iscoms do not create the adverse reactions associated with Quil A.

EP-A-231 039 indicates that the presence of antigen is not necessary for formation of the basic iscom structure, hereafter referred to as the iscom "matrix", it being possible to form the matrix from a sterol, such as cholesterol, a phospholipid, such as phosphatidyl-ethanolamine, and a glycoside such as Quil A. However, there was no disclosure that such matrices were useful as adjuvants; they were disclosed only in passing to show that iscom-like structures could be formed without the presence of an antigen. We have now found that such empty matrices can be used to provide an adjuvant function by addition to bacterial and mycoplasma antigens which do not form part of the iscom matrix.

Moreover, in all three of the cited prior art documents, when a water-insoluble antigen was used, this was solubilised with a detergent and then, after mixing with the glycoside etc, the detergent was removed, for example by dialysis, to cause formation of the iscoms. We have now found that iscoms will form in the presence of detergent and that this removal step is unnecessary. Iscom matrices may be formed in a similar manner.

One aspect of the invention provides a vaccine for human or animal use comprising (1) an antigen associated with a bacterium or mycoplasma and (2) an iscom matrix, the said antigen not being incorporated in the iscom.

The invention also encompasses a process for preparing a vaccine for human or animal use, comprising (1) forming a complex of a glycoside, a sterol and, optionally, a phospholipid, and (2) admixing the said complex with an antigen associated with a bacterium or mycoplasma.

By "animal", we mean non-human vertebrate, preferably a mammal such as a cow, pig, sheep, goat, horse, dog or cat.

The vaccine may comprise one or more diluents and carriers of a conventional nature. These will normally be added in or after step (2) of the process.

By "antigen associated with a bacterium or mycoplasma" we mean any entity capable of producing a protective antibody or cell-mediated immunological response against the bacterium or mycoplasma in a vertebrate exposed to the antigen. The antigen may be all or part of a protein, glycoprotein, glycolipid, polysaccharide or lipopolysaccharide which is associated with the bacterium or mycoplasma, or it may be a polypeptide or other entity which mimics all or part of such a protein, glycoprotein, glycolipid, polysaccharide or lipopolysaccharide. Because the antigen is not incorporated in the iscom matrix during formation of the latter, it is not necessary for it to have a hydrophobic region or to be linked to a hydrophobic group, as in prior art procedures. The bacterium or mycoplasma itself need not be present in the vaccine but it can reduce the cost of the vaccine if whole cells, for example of Haemophilus pleuropneumoniae , are used instead of extracts.

By "mycoplasma", we include the closely related organisms known as ureaplasmas and acholeplasmas.

Bacterial diseases include those caused by Mycobacterium (e.g. M. tuberculosis & M. bovis ), Clostridium (e.g. C. welchii ), Rickettsia, Spirochaetes (e.g. Treponema pallidum or Leptospira pomona ), Escherichia (e.g. E. coli ), Staphylococci (e.g. S. aureus ), Haemophilus (e.g. H. influenzae and H. pleuropneumoniae ), Bordetella, (e.g. B. pertussis ), Vibrio (e.g. V. cholerae ), Salmonella (e.g. S. typhi and S. paratyphi ), Streptococci (e.g. S. agalactiae ), Neisseria (e.g. N. gonorrhoea ), Pasteurella (e.g. P. multocida ), Legionella (e.g. L. pneumoniae ), Chlamydia (e.g. C. psittaci ), Pseudomonas (e.g. P. malliae ),

Actinobacillus (e.g. A. pleuropneumoniae ), Campylobacter (e.g. C. jejuni ), Listeria (e.g. L. monocytogenes ), Brucella (e.g. B. abortus ), Corynebacterium (e.g. C. diptheriae ), Yersinia (e.g. Y. pseudotuberculosis ), Pneumococci (e.g. Streptoccus pneumoniae ), Bacillus (e.g. B. anthracis ), Klebsiella (e.g. K. pneumoniae ), Shigella (e.g. S. dysenteriae ) and Actinomycetes (e.g. Nocardia asteroides ).

Preferably, the bacterium is a Mycobacterium (e.g. M. tuberculosis ), Clostridium (e.g. C. welchii ), Rickettsia, Spirochaetes (e.g. Treponema pallidum ), Escherichia (e.g. E. coli ), Staphylococci (e.g. S. aureus ), Haemophilus (e.g. H. influenzae and H. pleuropneumoniae ), Bordetella, (e.g. B. pertussis ), Vibrio (e.g. V. cholerae ), Salmonella (e.g. S. typhi and S. paratyphi , Streptococci (e.g. S. agalactiae ), Neisseria (e.g. N. gonorrhoea ), Pasteurella (e.g. P. multocida ), Legionella (e.g. L. pneumoniae ), Pseudomonas (e.g. P. malliae ), Actinobacillus (e.g. A. pleuropneumoniae ), Campylobacter (e.g. C. jejuni ), Listeria (e.g. L. monocytogenes ). Particular antigens include the adherence factor in Coli, e.g. pili K 88, porin protein in e.g. Salmonella and outer membrane proteins from B. pertussis and Neisseria meningitidis .

Preferably, the antigen is one which will not form a conventional iscom, i.e. as described in EP-A-109 942 and EP-A-180 564.

Vaccination of pigs against H. pleuropneumoniae is particularly preferred.

Mycoplasmas of veterinerary or medical interest include Mycoplasma bovis , M. gallisepticum , M. agalactiae , M. hyopneumoniae , M. pneumoniae , M. synoviae , M. arthritidis , M. capricolium , M. dispar , M. hominis , M. mycoides subs. capri , M. orale , M. oripneumoniae , M. pulmonis , M. cynos , M. hyorhinis , M. mycoides , M. salvarium and M. fermentans .

Although the antigen is not incorporated in the iscom matrix in the previous way, namely as part of the iscom structure itself, it may nevertheless be present on the outside of the iscom matrix or it may subsequently be integrated into the iscom matrix. Alternatively, it may be present entirely separately in solution or dispersion.

The iscom matrix may be prepared in the way disclosed in EP-A-0 231 039, namely by mixing together solubilised sterol, glycoside and (optionally) phospholipid and then removing the solubilising agent. Alternatively, the removal of the solubilising agent can be omitted. An electron micrograph of iscoms obtained from Quil A, cholesterol and phosphatidylethanolamine is depicted in Figure 1 of EP-A-0 231 039 (magnification 116.000 x). If phospholipids are not used, two dimensional structures are formed. An example of such structures is depicted in Figure 2 of EP-A-0 231 039. This figure is an electron micrograph (magnification 146.000 x) of structures obtained from Quil A and cholesterol. The term "iscom matrix" is used to refer to both the 3-dimensional and 2-dimensional structures.

The glycosides to be used in the process according to the invention may be the same as those mentioned in EP-A 0 109 942 and EP-A-0 231 039. Generally the glycosides are glycosides showing amphipathic properties and comprise hydrophobic and hydrophilic regions in the molecule. Preferably saponins are used, especially the saponin extract from Quillaja saponaria Molina in the first place DQ-extract prepared according to K. Dalsgard: Saponin Adjuvants. Bull. Off. Int.. Epiz. 77 & 7-8) 1289-1295 (1972) and Quil A, also prepared according to K. Dalsgaard: Saponin Adjuvants III Archiv für die gesamte Virusforschung 44 , 243-254 (1974). Other preferred saponins are aescine from Aesculus hippocastanum (T Patt and W Winkler: Das therapeutisch wirksame Prinzip der Rosskaatanie ( Aesculus hippocastanum ) Arzneimittelforschung 10 (4) 273-275 (1960) and sapoalbin from Gypsophilla struthium (R Vochten. P. Joos and R Ruyssen: Physicochemical properties of sapoalbin and their relation to the foam stability. J. Pharm. Belg. 42 213-226 (1968)). The use of Quil A is especially preferred.

In the process according to the invention the glycosides are used in at least the critical micelle-forming concentration. In the case of Quil A this concentration is about 0.03% by wt.

Generally, the molar ratio of glycoside (especially when it is Quil A) to sterol (especially when it is cholesterol) to phospholipid is 1:1:0-1, ± 20% (preferably no more than ± 10%) for each figure. This is equivalent to a weight ratio of about 5:1 for the Quil A: cholesterol.

The sterols used in the process according to the invention may be known sterols of animal or vegetable origin, such as cholesterol, lanosterol, lumisterol, stigmasterol and sitosterol. Preferably, cholesterol is the sterol used in the process according to the invention.

It is preferred for a phospholipid to be used, so that 3-dimensional iscoms are formed. Suitable phospholipids include phosphatidylcholine and phosphatidylethanolamine.

A second aspect of the invention provides a process for making an immunogenic complex comprising a water-insoluble antigen, the process comprising solubilising the antigen with a solubilising agent, admixing the solubilised antigen, a glycoside, a sterol and, optionally, a phospholipid and forming an iscom substantially without removal of the solubilising agent.

Thus, there is no need for the solubilising agent to be removed by ultrafiltration, dialysis, ultracentrifugation or chromatographic methods in order for the iscoms to be formed.

The antigen is as defined above, except that it may be associated with fungi, parasites such as protozoa and helminths or viruses instead of just mycoplasmas or bacteria.

Fungi associated with disease include: Candida spp., Cryptococcus spp., Aspergillus spp., Microsporum spp., Trichophyton spp. Epidermophyton spp. and Dermatophilus spp.

Parasites associated with disease include those listed in EP-A-109 942.

Viruses associated with disease include those listed in EP-A-109 942. Many of these have envelopes from which suitable antigens can be extracted. Particular viruses include equine influenza virus, equine herpes virus, bovine viral diarrhoea virus, coronavirus, parvovirus, feline leukaemia virus, feline immunodeficiency virus and equine viral arteritis virus.

The solubilising agent may be any of those mentioned in EP-A-0 109 942 or EP-A-0 231 039, for example a detergent, urea or guanidine.

Generally, a non-ionic, ionic or zwitter-ionic detergent or a cholic acid based detergent, such as sodium desoxycholate, can be used for this purpose. Preferably, the detergent used is octylglucoside, nonyl N-methyl glucamide or decanoyl N-methyl glucamide but alkylphenyl polyoxyethylene ethers are also suitable, especially a polyethylene glycol p-isooctylphenylether having 9 to 10 oxyethylene groups which is commercialized under the trade name Triton X-100R.

The process according to the invention may be used for the preparation of immunogenic complexes from antigenic proteins or peptides which show amphipathic properties, for example those described in EP-A-109 942.

Suitable methods for isolation of antigens and linking to hydrophobic groups are described in EP-A-109 942.

In the process according to the second aspect of the invention the dissolved or solubilized antigen is generally contacted with a solution containing the glycoside in at least the critical micelle-forming concentration, a sterol, and optionally a phospholipid.

If desired, the solutions of the immunogenic complexes obtained may be lyophilized. The lyophilized preparations may then be reconstituted before use by addition of water.

A third aspect of the invention provides a process for making an iscom matrix which process comprises solubilising a sterol, and optionally a phospholid, with a solubilising agent and mixing with a solution of the glycoside, without removal of the solubilising agent.

The solubilising agent, sterol, phospholid and glycoside are as hereinbefore defined.

Further, the invention relates to a pharmaceutical composition containing immunogenic complexes prepared by means of the first or second aspects of the invention. These properties may be obtained by preparing the immunogenic complexes in a form suitable for parenteral administration, preferably injectable administration and, in particular, for injection by the subcutaneous or intra-muscular routes. Generally, the pharmaceutical compositions contain the immunogenic complexes in an aqueous, physiologically acceptable medium, which, if desired, contains a buffer and/or a salt such as sodium chloride for adaptation of the osmotic pressure. These procedures are all well known in the art.

The following non-limiting examples illustrate various preferred aspects of the invention.

### EXAMPLES 1-6: Empty Iscom Matrix Preparation

Reagents Required

Phosphate Buffered Saline (Dulbecco A, Oxoid)
Lipid Mix (see below)
10% w/v solution of Quil A (Superfos) in sterile distilled water.

Preparation of Lipid Mix (10ml)

Materials:

Chloroform (analar), Cholesterol (Grade I, from porcine liver, Sigma), Lβphosphatidyl choline (Sigma), Mega 9 or 10 (Sigma) or Triton (Mega 9 is nonyl N-methyl glucamide, whereas Mega 10 is decanoyl N-methyl glucamide) and Sterile distilled water

Method

(a) Prepare 20% w/v of Mega 10 in sterile distilled water: ie weigh out 2g Mega 10 and dissolve in 10ml of water. (b) Weigh 50mg cholesterol and add to 50mg of phosphatidyl choline. (c) When the cholesterol has dissolved in the phosphatidyl choline add 2ml of chloroform. (d) Add the 10ml of 20% w/v Mega 10. The mixture will be milky-white in appearance. This will raise thevolume to 12ml. However, the chloroform will evaporate upon stirring and the final 10ml volume will be achieved. (e) Stand on a magnetic stirrer in the hot room (37°C), with the lid of the container removed. Upon continued stirring, the mixture will become more mobile and clear. (f) Store at room temperature.

## EXAMPLE 1

Protocol:

Place 20ml of PBSA into a sterile universal flask. Add 100μl of a 10% solution of Quil A. Add 400μl of the lipid mix. Whirlymix the solution at full speed for 10 seconds. Sterilise solution via passing through a 0.2μm filter (Millipore). Decant small volume for Electron microscopy studies. Store at +4°C.

## EXAMPLE 2

The method of Example 1 was followed, but with the following variants. Place 20ml of PBSA into a sterile universal flask. Add 200μl of a 10% solution of Quil A. Add 800μl of the lipid mix. Whirlymix the solution at full speed for 10 seconds. Sterilise through a 0.2μm filter. Decant a small volume for electron microscopy studies. Store at +4°C.

## EXAMPLE 3

20ml PBSA was added to an Amicon stirred cell with a 30K Mwt cut-off membrane. To this was added 800μl of the lipid mix plus 200μl of Quil A (10% solution). The solution was concentrated to 5ml and 100ml PBSA added. The above step was repeated x2. The 20ml contents were filtered through a 0.2μm filter labelled and stored at +4°C.

## EXAMPLE 4

20ml PBSA was added to an Amicon stirred cell with a30K Mwt cut-off membrane in place. 400μl of the lipid mix plus 100μl of a 10%Quil A solution was added. The above solution was concentrated to 5ml and resuspended to 100ml with PBSA. The above step was repeated x2. The final 20ml contents of the cell were then filter sterilised through a 0.2μm filter, labelled and stored at +4°C.

## EXAMPLE 5

20ml PBSA was added to an Amicon stirred cell, a 30K Mwt cut-off membrane being in place. 200μl of the lipid mix plus 50μl of a 10% Quil A solution was added. The above solution was concentrated to 5ml and resuspended in 100ml PBSA. The above step was repeated x2. The final 20ml prep of the cell was then filtered through a 0.2μm filter, labelled and stored at +4°C.

## EXAMPLE 6

5

20ml PBSA was added to an Amicon stirred cell (30K Mwt cut-off membrane). 100μl of the lipid mix plus 25μl of a 10% Quil Asolution was added. The above solution was concentrated to 5ml and resuspended with 100ml PBSA. The above step was repeated x2. The final 20ml contents of the cell were filter sterilised through a 0.2μm filter, labelled and stored at +4°C.

In electron microscopy studies, these iscom matrices appeared to be less numerous than in the other examples.

## EXAMPLE 7: Mouse Studies: Efficacy

Female mice of an inbred strain (Balb/C) and of the same age (6-8 weeks) were used. The mice were obtained from the barrier maintained (SPF) unit of Harlan-Olac (Bicester). The National Cell Type Collection strain of Mycoplasma hyopneumoniae was used throught this study. The organism was cultured in a modification of Friis medium. The stock cultures were maintained at -70°C. Each suspension for vaccine incorporation was prepared by inoculating 40ml of broth with 4ml of the stock culture at pH 7.4 and then incubating at 37°C for 3 days or until the pH dropped to 6.8. By this time the organism had reached the log phase of growth. The cultures were inactivated using binary ethylene imine, concentrated using tangential flow ultrafiltration, and washed with phosphate buffered saline (PBS). The concentration of antigen was adjusted such that all vaccines contained 10μg total protein per 0.1ml mouse dose of vaccine.

The iscom preparations of Examples 3 to 6 were used as follows.

Eight groups of six mice were immunised on two occasions, each mouse receiving 0.1ml of the following preparations.

| Grou-p | Vaccinate |
|---|---|
| A | 10μg M. hyopneumoniae alone |
| B | 10μg M. hyopneumoniae adjuvanted with Freund's Complete |
| C | 10μg M. hyopneumoniae with iscom matrix containing 10μg QA/mouse dose |
| D | 10μg M. hyopneumoniae with iscom matrix containing 5μg QA/mouse dose |
| E | 10μg M. hyopneumoniae with iscom matrix containing 2.5μg QA/mouse dose |
| F | 10μg M. hyopneumoniae with iscom matrix containing 1.25μg QA/mouse dose |
| G | 0μg M. hyopneumoniae with iscom matrix containing 5μg QA/mouse dose |
| H | 10μg M. hyopneumoniae + $10^7$ cfu H. pleuropneumoniae with iscom matrix containing 5μg QA/mouse dose |
| QA = Quil A | |

Groups of mice were pre-bled via the tail vein prior to primary immunisation and then fourteen days later. Twenty-eight days after the primary immunisation the animals received a further vaccination and were bled 7 days later and finally 28 days post secondary. Serum was separated from the blood on the day of collection and stored at -70°C for analysis by ELISA. Mice were observed following vaccination for any adverse local and systemic reactions.

## Results

The results of the experiments designed to assess the adjuvant effect of Quil A in an iscom preparation where the antigen is external to the preparation are shown below in Tables 1 and 2.

The results clearly demonstrate that the ISCOM preparation with the highest protein: Quil A ratio of

10μg: 10μg/mouse dose produces the greatest response to the vaccine, giving a response as good as if not better than the Freund's adjuvanted vaccine.

Groups of mice receiving iscom vaccines containing less than 10μg of Quil A per mouse dose performed no better, in producing a response to the antigen, than the unadjuvanted antigen alone.

No vaccine reactions either local or systemic were noted in any mice receiving iscom vaccine or with antigen alone. In mice immunised with Freund's, small granulomas approximately 3mm were noted at the immunisation point but as with all the other mice no signs of systemic disturbances were noted.

Table 1 -

| Anti M. hyopneumoniae response in mice as measured by the INDIRECT ELISA | | |
|---|---|---|
| Groups/Time | Pre Vaccination | 28DP2 ° |
| A | 0.023 | 0.184 |
| B | 0.019 | 0.126 |
| C | 0.019 | 0.203 |
| D | 0.019 | 0.043 |
| E | 0.019 | 0.083 |
| F | 0.032 | 0.029 |
| G | 0.042 | 0.052 |
| H | 0.040 | 0.150 |
| Standard Deviations omitted DP2° - days post secondary vaccination | | |

## EXAMPLE 8: Mouse Studies: Evaluation of Empty iscom matrix as an adjuvant for Haemophilus pleuropneumoniae vaccine in mice

Mice were immunised with vaccines containing whole inactivated H. pleuropneumoniae bacteria and iscom matrix formulated with varying levels of Quil A. Mice immunised twice with a vaccine containing 10μg Quil A/mouse gave a greater antibody response than mice immunised with equivalent numbers of bacteria in Freund's adjuvant. Lower doses of Quil A had little apparent adjuvant effect. No adverse local or systemic effects were observed following administration of any vaccine containing iscom matrix.

The NCTC reference strain of H. pleuropneumoniae serotype 3 (Strain 1421) was grown to log phase growth in Yeast extract medium supplemented with B NAD. The culture was inactivated with 0.2% formalin and washed three times with phosphate buffered saline (PBS) to yield a washed, inactivated whole cell antigen preparation. The antigen was standardised by enumeration of bacteria using a Neubaumer chamber with modified Thoma rulings and was stored in PBS at 4° C until use.

Groups of mice, six per group, were immunised with H. pleuro-pneumoniae alone, H. pleuropneumoniae in FIA, or H. pleuropneumoniae together with iscom matrix containing different levels of Quil A, as detailed below. A final group received both H. pleuropneumoniae and M. hyopneumoniae antigens together with iscom matrix to investigate possible interaction between the two antigens. Mice received two subcutaneous injections of 0.1 ml of the appropriate vaccine on two occasions with a 3 week interval between immunisations.

| Immunisation of mice | |
|---|---|
| Group | Vaccination |
| 1 | None |
| 2 | Hpl alone |
| 3 | Hpl in FIA |
| 4 | Hpl with iscom matrix containing 10μg QA/per mouse dose of vaccine |
| 5 | Hpl with iscom matrix containing 5μg QA/per mouse dose of vaccine |
| 6 | Hpl with iscom matrix containing 2.5μg QA/per mouse dose of vaccine |
| 7 | Hpl with iscom matrix containing 1.2μg QA/per mouse dose of vaccine |
| 8 | Hpl with iscom matrix containing 5μg QA/per mouse dose of vaccine and 10μg/per mouse dose Mycoplasma hyopneumoniae antigen. |

Following primary vaccination there was little antibody response in any group. Following secondary vaccination there was a rapid and pronounced rise in antibody levels in 2 out of the 8 groups immunised. The greatest response was seen in mice immunised with bacteria together with iscom matrix containing 10μg/mouse Quil A which had a mean peak OD of 1.24 (± 0.17) Units of Optical Density (UOD) when measured one week after secondary immunisation. Mice immunised with bacteria and FIA had a similar but slightly smaller antibody response with a mean peak OD of 1.11 (± 0.08) UOD on the same occasion. Mice immunised with bacteria alone, bacteria with lower doses of Quil A, or bacteria together with M. hyopneumoniae antigen all had similar, lower, antibody responses with peak values of between 0.43 and 0.63 UOD when measured one month after secondary immunisation.

Table 2

| Anti - H. pleuropneumonia e response, as measured by indirect ELISA | | |
|---|---|---|
| | Pre-vaccination | 28 DP2 |
| A | 0.87 | 0.189 |
| B | 0.170 | 0.428 |
| C | 0.185 | 1.002 |
| D | 0.135 | 1.161 |
| E | 0.131 | 0.626 |
| F | 0.164 | 0.645 |
| G | 0.119 | 0.620 |
| H | 0.227 | 0.480 |

**EXAMPLE 9: Pig Studies**

Thirty Landrace-cross piglets were obtained at three weeks of age from a herd known to be free of all clinical and serological evidence of infection with H. pleuropneumoniae and M. hyopneumoniae . Piglets were maintained in isolation from all other stock and were fed ad libertum with a standard weaner/grower diet.

A low passaged (less than 10) field isolate (SVA3) of M. hyopneumoniae was cultured to log phase of growth in Friis medium.

To 20 ml of sterile PBSA was added 400μl of a 10% Quil A solution and 1600μl of lipid mix. The mixture was then whirly-mixed for 20 seconds at maximum speed followed by filtration through a 0.45μm filter. To 10 ml of this ISCOM mix was added 10 ml of 4 mg/ml of M. hyopneumoniae antigen to yield an ISCOM mix containing 2 mg/ml of antigen and 1 mg/ml of Quil A.

A group of five pigs were immunised with 1 ml of ISCOM matrix containing 2 mg/ml M. hyopneumoniae whole cell antigen. A further five received 1 ml of 2 mg/ml M. hyopneumoniae adjuvanted with Freunds. The final 5 pigs received PBSA only.

Piglets received their first vaccination at 3 weeks of age by deep intramuscular injection of 1 ml of the appropriate vaccine into the deep muscles of the left side of the neck. A second similar injection was given 6 weeks later into the right side of the neck. Two weeks after the second immunisation, when they were 11 weeks of age, the piglets were experimentally challenged. Piglets were killed approximately 2 weeks after challenge for post-mortem examination for lung pathology.

### a) Clinical Reactions to Vaccination

No adverse clinical signs were observed in any pigs following primary or secondary vaccination. No significant rises in temperature after primary or secondary immunisation occurred in any group. The greatest rise in temperature, of 1.1 ± 0.23°C, was observed in piglets immunised with M. hyopneumoniae antigen in Freund's Incomplete adjuvant after the secondary immunisation. In general, mean group temperatures rose by approximately 0.3°C which was insufficient to cause clinical signs of pyrexia. Vaccines which contained M. hyopneumoniae antigens were not noticeably more pyrogenic than those which did not.

No local reactions were visible or palpable at the sites of injection of any vaccine on any occasion examined.

No differences between the weight gain of the piglets was observed.

### b) Clinical Reactions to Injection

Clinical reactions following infection were not apparent. The experimental challenge did not cause pyrexia. Pigs remained clinically normal for the two weeks following challenge. Weight gains over this period and feed intakes showed no significant difference between groups.

### c) Measurement of Serum Antibody

Following primary vaccination only the group receiving M. hyopneumoniae adjuvanted with Freund's demonstrated an antibody response. However, following a second immunisation there was a pronounced rise in antibody levels in all animals receiving M. hyopneumoniae vaccines. The serum M. hyopneumoniae antibody responses as measured by ELISA OD (@ 492 nm) prior to challenge were as follows:

| | |
|---|---|
| for animals receiving ISCOM vaccine | 0.6 ± 0.156 |
| for animals receiving Freund's adjuvanted vaccine | 1.7 ± 0.089 |

An anti M. hyopneumoniae antibody response (OD @ 492 nm = 0.6 ± 0.156) was also observed in animals receiving ISCOM matrix vaccines.

There was no further significant rise in antibody levels in any of the groups following infection.

### d) Local Reactions to Vaccination

The most severe reactions were observed in the piglets which were immunised with M. hyopneumoniae antigen in Freund's adjuvant. Reactions consisted of multiple dispersed caseous granulomata and were characteristic of reactions to Freund's adjuvants in the pig. No lesions were observed in pigs receiving ISCOM vaccines.

## EXAMPLE 10: Sheep Studies

Blackface x Swaledale ewes were acquired from a farm known to have been free of OEA for at least 4 years. The 5-6 year old animals were prescreened before use for antibodies to Chlamydia psittaci and Toxoplasma gondii . The ewes were maintained in isolation from other stock and were fed hay and concentrates at levels appropriate to their physiological and nutritional status.

### 1. Vaccines

OEA strains A22 and S26/3 of Chlamydia psittaci were grown and subsequently semi-purified. The initial culture volume of each strain of 10L was concentrated to 1L in this process to give "10X Antigen".

An Iscom vaccine (IV) was prepared as follows: (a) 50 ml of 10X antigen was treated with 100μl of stock (50%) glutaraldehyde for 15 min at 4°C. (b) 50 ml of filter-sterilized 0.15M glycine in PBS was added and the suspension incubated for 5 min at 4°C. (c) The suspension was centrifuged at 17000g for 45 min at 4°C, then the pellet washed twice with sterile PBS pH 7.2. (d) The pellet was resuspended in 50 ml PBS. (e) Thiomersal (1%; sterile filtered), 0.5 ml, was added to give a final concentration of 0.01%.

To 100 ml of sterile PBSA was added 50μl of a 10% Quil A solution and 2 ml of lipid mix. The mixture was stirred under 9 psi pressure (62 kN.m$^{-2}$) and concentrated to 20 ml volume. Sterile PBSA was added to make the volume up to 200 ml and the volume then reduced to 20 ml. This was repeated twice. The final volume was filtered through a 0.45μm filter.

The antigen preparation was added to the Iscom mix to give an antigen concentration of 0.25X antigen/ml.

One oil-based (OBV) and one Alhydrogel-adsorbed aqueous vaccines (AAV) were prepared as controls to give 1X antigen/ml and 0.25 X antigen/ml respectively.

The experiment comprised 5 groups and a total of 106 ewes. Groups 1, 2 and 3 were vaccinated with OBV, AAV and IV respectively. Groups 4 and 5 constituted the controls, being infected/unvaccinated and uninfected/unvaccinated respectively.

The OBV vaccine was administered once only as a 1 ml dose given 9 weeks before tupping. The other 2 vaccines were administered twice, 9 weeks and 3 weeks before tupping, each injection comprising a 2 ml dose (all vaccines delivered a payload of 4μg Chlamydial protein).

All vaccines were injected subcutaneously at an identified, shorn site on the right side of the neck.

Ewes of Groups 1, 2, 3 and 4 were challenged with live chlamydia at 85-90 d.g. Group 5 remained untreated.

At lambing or abortion, placentas, if available, were taken for sampling. The condition of the lamb at this time (live, moribund or dead), was noted. No attempt was made to keep alive sickly lambs, apart from the provision of colostrum.

The presence or otherwise of OEA lesions in placentas was noted and an estimate made of their extent. Placentas (or vaginal swabs) were examined for chlamydial bodies by staining by the modified Ziehl-Neelsen method, and cultivated in BHK21 cells treated with 5-iododeoxyridine.

### Results

The results showed that the ISCOM vaccine provided protection against the Chlamydial infection that was at least as good as that provided by the conventional vaccines used in the experiment.

## EXAMPLE 11: Equine Influenza Virus Iscom Preparation

### Protocol:

Place 20 ml of PBSA into a sterile universal flask. Add 100μl of a 10% solution of Quil A. Add 400μl of the lipid mix. Add ERIK-14 equine flu antigen component to a final concentration of 5μg/ml. Whirlymix at full speed for 20 seconds. Sterilise solution via passing through a 0.2μm filter (Millipore). Decant small volume for electron microscopy studies (iscom structures observed) and bacterial contamination check. Store at +4°C.

**Claims**

1. A vaccine for human or animal use comprising (1) an antigen associated with a bacterium or mycoplasma and (2) an iscom matrix, the said antigen not being incorporated in the iscom.

2. A vaccine according to Claim 1 wherein the antigen is associated with one of the following species, genera or classes of organism: Mycobacterium, Clostridium, Rickettsia, Spirochaetes, Escherichia, Staphylococci, Haemophilus, Bordetella, Vibrio, Salmonella, Streptococci, Pasteurella, Legionella, Chlamydia, Pseudomonas, Actinobacillus, Campylobacter, Listeria, *Mycoplasma bovis, M. gallisepticum, M. agalactiae, M. hyopneumoniae, M. pneumoniae, M. synoviae, M. arthritidis, M. capricolium, M. dispar, M. hominis, M. mycoides subs. capri, M. orale, M. oripneumoniae, M. pulmonis, M. cynos, M. hyorhinis, M. mycoides, M. salvarium* and *M. fermentans*, or the antigen comprises the adherence factor in Coli, porin protein or outer membrane proteins from *B. pertussis* or *Neisseria meningitidis*.

3. A vaccine according to Claim 1 or 2 wherein the iscom matrix comprises a phospholipid and a solubilising agent.

4. A vaccine according to any one of the preceding claims wherein the iscom matrix comprises a solubilising agent selected from octylglycoside, nonyl N-methyl glucamide, urea and guanidine.

5. A vaccine according to any one of the preceding claims wherein the iscom matrix comprises a sterol other than cholesterol.

6. A process for preparing a vaccine for human or animal use, comprising (1) forming a matrix of a glycoside and a sterol and (2) admixing the said complex with an antigen associated with a bacterium or mycoplasma.

7. A process for preparing a vaccine for human or animal use, comprising (1) forming an iscom matrix of a glycoside and a sterol and (2) admixing the said iscom matrix with an antigen associated with a bacterium or mycoplasma, wherein either (i) a phospholipid and a solubilising agent are additionally used in step (1) or (ii) a solubilising agent selected from octylglycoside, nonyl N-methyl glucamide, urea and guanidine is used in step (1) or (iii) the said sterol is not cholesterol.

8. A process according to Claim 6 or Claim 7 conditions (ii) or (iii), wherein a phospholipid is additionally used in step (1).

9. A process according to Claim 7 or 8 wherein a solubilising agent is used in step (1) and is not removed prior to formation of the iscom matrix.

10. A process for making an immunogenic complex comprising a water-insoluble antigen, the process comprising solubilising the antigen with a solubilising agent, admixing the solubilised antigen, a glycoside and a sterol and forming an iscom substantially without removal of the solubilising agent.

11. A process according to Claim 10 wherein a phospholipid is additionally used in the said admixing step.

12. A process according to Claim 10 or 11 wherein the solubilising agent is sodium desoxycholate, octylglucoside, nonyl N-methyl glucamide, decanoyl N-methyl glucamide or a polyethylene glycol p-isooctyl-phenylether having 9 to 10 oxyethylene groups.

13. A process for making an iscom matrix, the process comprising admixing a glycoside, a sterol and a solubilising agent and forming an iscom matrix substantially without removal of the solubilising agent.

14. A vaccine for animal use comprising (1) an antigen associated with a bacterium or mycoplasma and (2) an iscom matrix, the said antigen not being incorporated in the iscom, wherein the animal is a pig or a sheep.

Claims for the following Contracting States: ES, GR

1. A process for preparing a vaccine for human or animal use, comprising (1) forming a matrix of a glycoside and a sterol and (2) admixing the said matrix with an antigen associated with a bacterium or mycoplasma.

2. A process according to Claim 1 wherein a phospholipid is additionally included in step (1).

3. A process according to Claim 2 wherein the phospholipid is phosphatidylcholine.

4. A process according to any one of Claims 1 to 3 wherein the glycoside is Quil A.

5. A process according to any one of Claims 1 to 4 wherein the sterol is cholesterol.

6. A process according to any one of Claims 1 to 5 wherein the antigen is associated with one of the following species, genera or classes of organism: Mycobacterium, Clostridium, Rickettsia, Spirochaetes, Escherichia, Staphylococci, Haemophilus, Bordetella, Vibrio, Salmonella, Streptococci, Pasteurella, Legionella, Chlamydia, Pseudomonas, Actinobacillus, Campylobacter, Listeria, *Mycoplasma bovis, M. gallisepticum, M. agalactiae, M. hyopneumoniae, M. pneumoniae, M. synoviae, M. arthritidis, M. capricolium, M. dispar, M. hominis, M. mycoides subs. capri, M. orale, mM. oripneumoniae, M. pulmonis,*

*M. cynos, M. hyorhinis, M. mycoides, M. salvarium* and *M. fermentans,* or the antigen comprises the adherence factor in Coli, porin protein or outer membrane proteins from *B. pertussis* or *Neisseria meninigitidis.*

7. A process according to any one of the preceding claims wherein step (1) comprises forming a matrix from a glycoside, a sterol, a phospholipid and a solubilising agent.

8. A process according to any one of the preceding claims wherein step (1) comprises forming a matrix from a glycoside, a sterol and solubilising agent selected from octylglycoside, nonyl N-methyl glucamide, urea and guanidine.

9. A process according to any one of the preceding claims wherein the sterol used in step (1) is other than cholesterol.

10. A process according to Claim 9 wherein the sterol is lanosterol, lumisterol, stigmasterol or sitosterol.

11. A process according to Claim 8, 9 or 10 wherein step (1) comprises forming a matrix of the glycoside, the sterol and a phospholipid.

12. A process according to any one of the preceding claims wherein the antigen is associated with *Mycoplasma hyopneumoniae.*

13. A process according to any one of the preceding claims wherein a solubilising agent is used in step (1) and is not removed prior to formation of the iscom matrix.

14. A process for making an immunogenic complex comprising a water-insoluble antigen, the process comprising solubilising the antigen with a solubilising agent, admixing the solubilised antigen, a glycoside and a sterol and forming an iscom substantially without removal of the solubilising agent.

15. A process according to Claim 17 wherein a phospholipid is additionally used in the said admixing step.

16. A process according to Claim 15 wherein the iscoms are formed other than in the course of ultrafiltration, dialysis, ultracentrifugation or chromatographic methods.

17. A process according to any one of Claims 14 to 16 wherein the solubilising agent is sodium desoxycholate, octylglucoside, nonyl N-methyl glucamide, decanoyl N-methyl glucamide or a polyethylene glycol p-isooctyl-phenylether having 9 to 10 oxyethylene groups.

18. A process for making an iscom matrix, the process comprising admixing a glycoside, a sterol and a solubilising agent and forming an iscom matrix substantially without removal of the solubilising agent.

19. A process according to any one of the preceding claims wherein the vaccine is for use on pigs or sheep.

European
Patent Office

EUROPEAN SEARCH
REPORT

Application Number

EP 90 30 9570

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 107, no. 24, 14th December 1987, page 354, abstract no. 223109w, Columbus, Ohio, US; M. TRUDEL et al.: "Vacoination of rabbits with a bovine herpesvirus type 1 subunit vaccine: adjuvant effect of ISC-OMs", & VACCINE 1987, 5(3), 239-43 * Abstract * | 1-14 | A 61 K 39/39 |
| D,A | EP-A-0 231 039 (DE STAAT DER NEDERLANDEN) | 1-14 | |

TECHNICAL FIELDS
SEARCHED (Int. Cl.5)

A 61 K

The present search report has been drawn up for all claims

| Place of search | Date of completion of search | Examiner |
|---|---|---|
| The Hague | 15 November 90 | BRINKMANN C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document